(19) Europäisches Patentamt
European Patent Office
Office européen des brevets

(11) Veröffentlichungsnummer: **0 205 402**
**B1**

(12) **EUROPÄISCHE PATENTSCHRIFT**

(45) Veröffentlichungstag der Patentschrift: **23.05.90**

(51) Int. Cl.$^5$: **C 07 D 303/28,** C 08 G 59/20

(21) Anmeldenummer: **86810234.4**

(22) Anmeldetag: **02.06.86**

# (54) 2,6-Disubstituierte 4-Epoxypropylphenylglycidylether und deren Verwendung.

(30) Priorität: **06.06.85 CH 2398/85**

(43) Veröffentlichungstag der Anmeldung:
**17.12.86 Patentblatt 86/51**

(45) Bekanntmachung des Hinweises auf die Patenterteilung:
**23.05.90 Patentblatt 90/21**

(84) Benannte Vertragsstaaten:
**CH DE FR GB IT LI NL**

(56) Entgegenhaltungen:
**FR-A-1 173 530**
**FR-A-1 185 580**

(73) Patentinhaber: **CIBA-GEIGY AG**
**Klybeckstrasse 141**
**CH-4002 Basel (CH)**

(72) Erfinder: **Monnier, Charles E., Dr.**
**Ch. du Verger 16**
**CH-1752 Villars-sur-Glâne (CH)**
Erfinder: **Zahir, Sheik Abdul-Cader, Dr.**
**Elsternstrasse 12**
**CH-4104 Oberwil (CH)**
Erfinder: **Eldin, Sameer H., Dr.**
**Route de Schiffenen 10**
**CH-1700 Fribourg (CH)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 205 402 B1

**Beschreibung**

Die vorliegende Erfindung betrifft neue 2,6-disubstituierte 4-Epoxypropylphenylglycidylether, solche 4-Epoxypropylphenylglycidylether enthaltende härtbare Gemische und deren Verwendung, z.B. zur Herstellung von gehärteten Produkten.

Aus der Literatur sind Glycidylether von ein- oder mehrkernigen Phenolen bekannt, die in einer oder beiden ortho-Stellungen zur 2,3-Epoxypropoxygruppe 2,3-Epoxypropylgruppen aufweisen (vgl. z.B. die frantäsisohe Patentschrift 1 185 580 und die britische Patentschrift 828.364). Diese Phenylglycidylether werden durch Epoxidierung der entsprechenden o-Allylphenole hergestellt, die ihrerseits durch Claisen-Umlagerung der Phenylallyläther erhalten werden. Dabei entstehen jedoch Isomerengemische bzw. Gemische aus 2-Allyl- und 2,6-Diallylphenolen.

Gegenstand der Erfindung sind neue hochreine 4-(2,3-Epoxypropyl)-2,6-disubstituierte Phenylglycidylether der Formel I

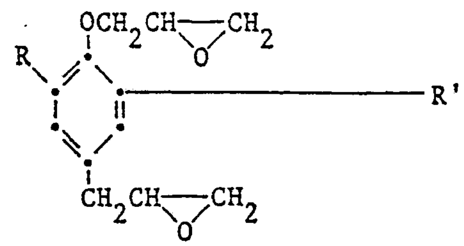

(I),

worin R und R′ unabhängig voneinander $C_{1-4}$-Alkyl, $C_{1-4}$-Alkoxy,

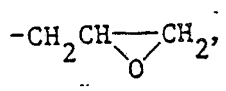

ein Halogenatom oder $C_{6-10}$-Aryl bedeuten.

Alkyl- und Alkoxysubstituenten R und R′ können geradkettig oder verzweigt sein. Beispiele solcher Gruppen sind: Methyl, Ethyl, n-Propyl, Isopropyl, n-Butyl, sek-Butyl, tert-Butyl, Methoxy, Ethoxy, n-Propoxy, n-Butoxy und sek-Butoxy. Als Arylgruppen R und/oder R′ kommen z.B. 1-Naphthyl, 2-Naphthyl und besonders Phenyl in Betracht. Stellen R und/oder R′ Halogenatome dar, so handelt es sich z.B. um Brom- oder Fluoratome und besonders um Chloratome.

Bevorzugt sind Verbindungen der formel I, worin R und R′ unabhängig voneinander $C_{1-4}$-Alkyl, $C_{1-2}$-Alkoxy, ein Halogenatom, besonders ein Chloratom, oder Phenyl bedeuten. Gemäss einer weiteren Bevorzugung haben R und R′ dieselbe Bedeutung; Besonders bevorzugt sind Verbindungen der Formel I, worin R und R′ je Methyl, tert-Butyl, Methoxy, Chlor oder Phenyl bedeuten. Ganz besonders bevorzugt sind Verbindungen der Formel I, worin R und R′ je Methyl darstellen.

Die Verbindungen der Formel I können auf an sich bekannte Weise hergestellt werden, z.B. indem man einen Allylphenylglycidylether der Formel II

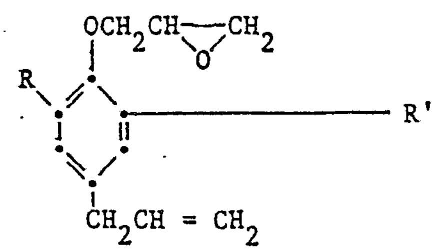

(II),

worin R und R′ unabhängig voneinander $C_{1-4}$-Alkyl, $C_{1-4}$-Alkoxy, ein Halogenatom oder $C_{6-10}$-Aryl darstellen, in Gegenwart einer Persäure epoxidiert.

Als Persäuren eignen sich vor allem organische Persäuren, wie z.B. Perameisensäure, Peressigsäure, Perbenzoesäure und Monoperphthalsäure.

Die organischen Persäuren können als solche eingesetzt oder in situ gebildet werden, beispielsweise aus aliphatischen oder aromatischen Carbonsäuren, Carbonsäureanhydriden, Carbonsäureestern, Säurechloriden oder Keten und Wasserstoffperoxid. Zur in-situ-Bildung der Persäuren verwendet man vorzugsweise aliphatische oder aromatische Mono- oder Dicarbonsäuren oder deren Anhydride, wie Ameisensäure, Essigsäure, Propionsäure, Bernsteinsäureanhydrid, Benzoesäure oder Phthalsäure, und Wasserstoffperoxid, gegebenenfalls unter Zusatz von säuren Katalysatoren, wie Schwefelsäure oder Alkalimetallsalzen. Die Epoxidierung der Verbindungen der Formel II wird bevorzugt in Gegenwart von vorgebildeter

oder in situ erzeugter Perameisensäure oder Peressigsäure durchgeführt. Gewünschtenfalls können auch anorganische Persäuren, wie Permolybdänsäure, Pervanadinsäure oder Perwolframsäure, eingesetzt werden. Das Epoxidierungsmittel (Persäure) wird zweckmässig in einer Menge von mindestens 1 Mol pro vorhandene Allylgruppe und vorzugsweise im Ueberschuss, z.B. einem 20—200%igen molaren Ueberschuss, verwendet.

Die Epoxidierung der Verbindungen der Formel II wird mit Vorteil in Gegenwart inerter organischer Lösungsmittel und gegebenenfalls unter Zusatz von Puffersubstanzen, wie Natriumacetat oder Natriumhydrogenphosphat, vorgenommen. Als Lösungsmittel eigen sich z.B. gegebenenfalls halogenierte aliphatische oder aromatische Kohlenwasserstoffe, wie Chloroform, Dichlormethan, Benzol, Toluol und Chlorbenzol, Ether wie Diethylether, Diisopropylether, Dioxan und Tetrahydrofuran, sowie Carbonsäurealkylester, wie Essigsäureethylester und -n-butyl-ester.

Halogenierte, besonders chlorierte aliphatische Kohlenwasserstoffe sind als Lösungsmittel bevorzugt; besonders bevorzugt ist Chloroform. Die Epoxidierungstemperaturen liegen im allgemeinen zwischen −10 und +100°C, bevorzugt zwischen 10 und 60°C.

Die Ausgangsprodukte der Formel II sind bekannt oder können nach an sich bekannten Methoden durch Umsetzung der entsprechenden 2,6-disubstituierten 4-Allylphenole mit Epihalogenhydrinen, besonders Epichlorhydrin, in Gegenwart von Katalysatoren hergestellt werden.

Die Verbindungen der Formel I sind hochreine Substanzen, die destilliert und umkristallisiert werden können und frei von Chlorid- und Alkalimetallionen sind. Sie fallen in sehr hohen Ausbeuten an.

Die Verbindungen der Formel I eignen sich zur Herstellung von gehärteten Produkten. Gegenstand der Erfindung sind daher auch härtbare Gemische enthalktend.

(a) eine Verbindung der Formel I und

(b) einen Härter für die Komponente (a).

Dabei können auch Gemische verschiedener Verbindungen der Formel I und/oder Härter verwendet werden.

Als Härter (b) eignen, sich an sich beliebige Epoxidharzhärter, wie z.B. Cyanamid, Dicyandiamid, Polycarbonsäuren, Polycarbonsäureanhydride, Polyamine, Polyaminoamide, Addukte aus Aminen und Polyepoxiden und Polyole.

Geeignete Polycarbonsäuren und ihre Anhydride sind z.B. Phthalsäureanhydrid, Tetrahydro- und Hexahydrophthalsäureanhydrid, Methyltetrahydrophthalsäureanhydrid, Endomethylentetrahydrophthalsäureanhydrid, Hexachlorendomethylentetrahydrophthalsäureanhydrid, Trimellitsäureanhydrid, Pyromellitsäuredianhydrid, Maleinsäureanhydrid, Bernsteinsäureanhydrid, Nonenylbernsteinsäureanhydrid, Dodecenylbernsteinsäureanhydrid, Polysebacinsäurepolyanhydrid und Polyazelainsäurepolyanhydrid sowie die zu den oben genannten Anhydriden gehörenden Säuren.

Als Beispiele von Polyaminen, die sich als Härtungsmittel eignen, seien aliphatische, cycloaliphatische, aromatische und heterocyclische Polyamine, wie Ethylendiamin, Propan-1,2-diamin, Propan-1,3-diamin, N,N-Diethylethylendiamin, Hexamethylendiamin, Diethylentriamin, Triethylentetramin, Tetraethylenpentamin, N-(2-Hydroxyethyl)-, N-(2-Hydroxypropyl)- und N-(2-Cyanethyl)diethylentriamin, 2,2,4- und 2,4,4-Trimethylhexan-1,6-diamin, m-Xylylendiamin, N,N-Dimethyl- und N,N-Diethylpropan-1,3-diamin, Bis(4-amino-cyclohexyl)methan, 2,2,-Bis(4-aminocyclohexyl)propan, 2,2-Bis(4-amino-3-methylcyclohexyl)-propan, 3-Aminomethyl-3,5,5-trimethylcyclohexylamin (Isophorondiamin), m- und p-Phenylendiamin, Bis(4-aminophenyl)-methan, Bis(4-aminophenyl)sulfon, Anilin-Formaldehydharze und N-(2-Aminoethyl)-piperazin genannt. Geeignete Polyaminoamide sind z.B. solche, die aus aliphatischen Polyaminen und dimerisierten oder trimerisierten ungesättigten Fettsäuren hergestellt sind.

Als Addukte aus Aminen und Polyepoxiden kommen z.B. Addukte aus aliphatischen oder cycloaliphatischen Diamine, wie 1,6-Hexamethylendiamin, 2,2,4- und 2,4,4-Trimethylhexan-1,6-diamin oder Isophorondiamin und bekannten Diglycidylethern in Betracht.

Als Polyolhärter (b) kommen vor allem ein- oder mehrkernige aromatische Polyole, einschliesslich Novolake, in Betracht, wie Resorcin, Hydrochinon, 2,6-Dihydroxytoluol, Pyrogallol, 1,1,3-Tris(hydroxyphenyl)propan, Bis(4-hydroxphenyl)methan, 2,2-Bis(4-hydroxyphenyl)propan, Bis(4-hydroxyphenyl)sulfon und 4,4′-Dihydroxybiphenyl sowie Novolake aus Formaldehyd oder Acetaldehyd und Phenol, Chlorphenol oder Alkylphenolen mit bis zu 9 C-Atomen im Alkyl, besonders Kresol- und Phenolnovolake.

Bevorzugte Härter sind Polycarbonsäureanhydride, wie Tetrahydro-, Hexahydro- und Methyltetrahydrophthalsäureanhydrid, sowie aromatische Polyamine, besonders Bis(4-aminophenyl)methan, Bis(4-aminophenyl)sulfon und m- oder p-Phenylendiamin.

Die Härter (b) werden in den in der Epoxidharztechnik üblichen Mengen eingesetzt, zweckmässig in solchen Mengen, dass auf ein Epoxidäquivalent etwa 0,7 bis 1,5 Aequivalente funktioneller Gruppen des Härters (b) kommen.

Die erfindungsgemässen Gemische können auch weitere übliche Zusätze enthalten, wor allem (c) Beschleuniger bzw. Härtungskatalysatoren, und/oder (d) weitere Epoxidharze.

Als Beschleuniger (c) können ebenfalls an sich bekannte Verbindungen verwendet werden. Als Beispiele seien genannt: Komplexe von Aminen, besonders tertiären Aminen, wie Monoethylamin, Trimethylamin und Octyldimethylamin, mit Bortrifluorid oder Bortrichlorid, tertiäre Amine, wie Benzyldimethylamin, Tri(dimethylaminomethyl)phenol, Hexamethylentetramin oder 1,6-Bis(dimethylamino)-hexan; Harnstoffderivate, wie N-4-Chlorphenyl-N′, N′-dimethylharnstoff (Monuron), N-3-Chlor-4-methylphenyl-N',N'-dimethylharnstoff (Chlortoluron), N-(2-Hydroxyphenyl)-N',N'-dimethylharnstoff und N-(2-Hydroxy-4-nitrophenyl)-N',N'-dimethylharnstoff, und gegebenenfalls substituierte Imidazole, wie Imidazol, Benzimidazol, 1-Methylimidazol, 3-Methylimidazol, 2-Ethyl-4-methylimidazol, 2-Vinylimidazol, 2-Phenylimidazol, 2-Phenyl-4-methylimidazol, 1-(2,6-Dichlorbenzoyl)-2-phenylimidazol und 1-(2,4,6-Trimethylbenzoyl)-2-phenylimidazol.

Tertiäre Amine, besonders Benzyldimethylamin, und Imidazole,besonders 2-Phenylimidazol, 3-Methylimidazol und 2-Ethyl-4-methylimidazol, sind als Beschleuniger (c) bevorzugt.

Als weitere Epoxidharze (d) kommen vor allem solche mit durchschnittlich mehr als einer an ein Heteroatom, z.B. an ein S- und vorzugsweise an ein O— oder N-Atom, gebundenen Gruppe der Formel III

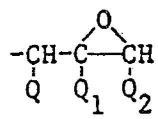

(III)

in Betracht, wobei Q und $Q_2$ je ein Wasserstoffatom und $Q_1$ ein Wasserstoffatom oder eine Methylgruppe bedeuten oder Q und $Q_2$ zusammen $—CH_2CH_2—$ oder $—CH_2CH_2CH_2—$ und $Q_1$ ein Wasserstoffatom bedeuten.

Als Beispiele solcher Harze seien Polyglycidyl- und Poly(β-methylglycidyl)ester genannt, die sich von aliphatischen, cycloaliphatischen oder aromatischen Polycarbonsäuren ableiten. Beispiels geeigneter Polycarbonsäuren sind Bernsteinsäure, Glutarsäure, Adipinsäure, Pimelinsäure, Korksäure, Azelainsäure, Sebacinsäure, dimerisierte oder trimerisierte Linolsäure, Tetrahydrophthalsäure, 4-Methyltetrahydrophthalsäure, Hexahydrophthalsäure, 4-Methylhexahydrophthalsäure, Phthalsäure, Iso- und Terephthalsäure.

Weitere Beispiele sind Polyglycidyl- und Poly(β-methylglycidyl)ether, die durch Umsetzung einer mindestens zwei alkoholische und/oder phenolische Hydroxylgruppen pro Molekül enthaltenden Verbindung mit Epichlorhydrin oder mit Allylchlorid und anschliessende Epoxidierung mit Persäuren erhalten werden.

Geeignete Polyole sind z.B. Ethylenglykol, Diethylenglykol, Poly(oxyethylen)glykole, Propan-1,2-diol, Poly(oxypropylen)glykole, Propan-1,3-diol, Butan-1,4-diol, Poly(oxytetramethylen)glykole, Pentan-1,5-diol, Hexan-2,4,6-triol, Glycerin, 1,1,1-Trimethylolpropan, Pentaerythrit und Sorbit; 1,3- und 1,4-Cyclohexandiol, Bis-(4-hydroxycyclohexyl)methan,2,2-Bis-(4-hydroxycyclohexyl)propan und 1,1,-Bis(hydroxymethyl)cyclohex-3-en; N,N-Bis(2-hydroxyethyl)anilin und 4,4'-Bis(2-hydroxyethylamino)diphenylmethan; Resorcin, Hydrochinon, Bis-(4-hydroxyphenyl)methan (Bisphenol F), 2,2-Bis(4-hydroxyphenyl)propan (Bisphenol A), 2,2-Bis(4-hydroxy-3,5-dibromphenyl)propan (Tetrabrombisphenol A), 1,1,2,2-Tetrakis(4-hydroxyphenyl)ethan, 4,4'-Dihydroxybiphenyl, Bis-(4-hydroxyphenyl)sulfon sowie Novolake aus Formaldehyd oder Acetaldehyd und Phenol, Chlorphenol oder Alkylphenolen mit bis zu 9 C-Atomen im Alkyl, besonders Kresol- und Phenol-Novolake.

Als Poly(N-glycidyl)-Verbindungen kommen durch Dehydrochlorierung von Umsetzungprodukten aus Epichlorhydrin und Aminen mit mindestens zwei Aminwasserstoffatomen erhaltene Produkte in Betracht. Geeignete Amine sind z.B. Anilin, n-Butylamin, Bis-(4-aminophenyl)methan, 1,3- und 1,4-Xylylendiamin, 1,3,- und 1,4-Bis(aminomethyl)cyclohexan und Bis(4-methylaminophenyl)methan. Triglycidylisocyanurat, N,N'-Diglycidylderivate von cyclischen Alkylenharnstoffen, wie Ethylenharnstoff und 1,3-Propylenharnstoff, oder Hydantoinen, wie 5,5-Dimethylhydantoin, sind weitere geeignete derartige Verbindungen.

Poly(S-glycidyl)-Verbindungen sind z.B. die Di-S-Glycidylderivate von Dithiolen, wie Ethan-1,2-dithiol und Bis-(4-mercaptomethylphenyl)ether.

Beispiele für Epoxidharze mit einer oder mehreren Gruppen der Formel III, in welcher Q und $Q_2$ zusammen eine Gruppe $—CH_2CH_2—$ oder $—CH_2CH_2CH_2—$ bedeuten, sind Bis(2,3-epoxycyclopentyl)ether, 2,3-Epoxycyclopenthylglycidylether, 1,2-Bis(2,3-epoxycyclopentyloxy)ethan, 3,4-Epoxy-6-methylcyclohexylmethyl-3',4'-epoxy-6'-methylcyclohexancarboxylat und 2-(3,4-Epoxy)cyclohexyl-5,5-spiro(3',4'-epoxy)cyclohexan-dioxan.

Ebenfalls einsetzbar sind Epoxidharze, in welchen die Epoxidgruppen an Heteroatome verschiedener Art gebunden sind, oder in denen einige oder sämtliche Epoxidgruppen mittelständig sind, wie beispielsweise das N,N,O-Triglycidylderivat des 4-Aminophenols, N-Glycidyl-N'(2-glycidyloxypropyl)-5,5-dimethylhydantoin, Vinylcyclohexendioxid, Limonendioxid, und Dicyclopentadiendioxid.

Besonders bevorzugt setzt man als Komponente (d) gegebenenfalls vorverlängerte Diglycidylether von zweiwertigen Phenolen, vor allem 2,2-Bis-(4-hydroxyphenyl)propan, 2,2-Bis(3,5-dibrom-4-hydroxyphenyl)-propan, Bis(4-hydroxyphenyl)methan, Bis(4-hydroxycyclohexyl)methan oder 2,2-Bis(4-hydroxycyclohexyl)propan, Polyglycidylether von Novolaken, oder tetraglycidyliertes 4,4'-Diaminodiphenylmethan ein. Ganz besonders bevorzugt sind gegebenenfalls vorverlängerte Diglycidylether von Bisphenol A, Tetrabrom-Bisphenol A oder Bisphenol F, Polyglycidylether von Phenol-Formaldehyd- oder Kresol-Formaldehyd-Novolaken, oder Gemische davon.

Bei Verwendung höherer Anteile an Epoxidharzen (d), z.B. in Mengen von bis 90 Gew.%, bezogen auf die Komponenten (a) und (d), können die Verbindungen der Formel I auch als reaktive Verdünner wirken.

Die Komponenten (b) und (c) werden in den üblichen wirksamen, d.h. für die Härtung der erfindungsgemässen Gemische ausreichenden Mengen eingesetzt. Das Verhältnis der Komponenten (a), (b), (c) und gegenbenenfalls (d) hängt von der Art der verwendeten Verbindungen, der erforderlichen Härtungsgeschwindigkeit und den im Endprodukt gewünschten Eigenschaften ab und kann vom Fachmann auf dem Gebiet der Epoxidharze-Härtung leicht ermittelt werden. Wenn das Härtungsmittel (b) ein Amin ist, werden normalerweise 0,75 bis 1,25 Aequivalente Aminwasserstoff pro 1 Epoxidäquivalent eingesetzt. Bei Polycarbonsäure- oder Polycarbonsäureanhydrid-Härten verwendet man gewöhnlich 0,4 bis 1,1 Aequivalente Carboxyl- bzw. Anhydridgruppen pro 1 Epoxidäquivalent. Bei der Verwendung von Polyphenolen als Härtungsmittel setzt man zweckmässig 0,75 bis 1,25 phenolische Hydroxylgruppen pro 1 Epoxidäquivalent ein. Beschleuniger (c) werden im allgemeinen in Mengen von 0,1 bis 5 Gewichtsprozent, bezogen die Epoxidharze (a) und gegenbenenfalls (d), verwendet.

Gewünschtenfalls kann man den härtbaren Gemischen zur Herabsetzung der Viskosität (weitere) reaktive Verdünner, wie z.B. Styroloxid, Butylglycidylether, 2,2,4-Trimethylpentylglycidylether, Phenylglycidylether, Kresylglycidylether oder Glycidylester von synthetischen, hochverzweigten, in der Hauptsache tertiären aliphatischen Monocarbonsäuren, zusetzen. Als weitere übliche Zusätze können die erfindungsgemässen Gemische ferner Weichmacher, Streck-, Füll- und Verstärkungsmittel, wie beispielsweise Steinkohlenteer, Bitumen, Textilfasern, Glasfasern, Asbestfasern, Borfasern, Kohlenstoff-Fasern, mineralische Silikate, Glimmer, Quarzmehl, Aluminiumoxidhydrat, Betonite, Kaolin, Kieselsäureaerogel oder Metallpulver, z.B. Aluminiumpulver oder Eisenpulver, ferner Pigmente und Farbstoffe, wie Russ, Oxidfarben und Titandioxid, Flammschutzmittel, Thixotropiemittel, Verlaufmittel ("flow control agents"), wie Silicone, Wachse und Stearate, die zum Teil auch als Formtrennmittel Anwendung finden, Haftvermittler, Antioxidantien und Lichtschutzmittel enthalten.

Die erfindungsgemässen Gemische finden z.B. Anwendung als Klebstoffe oder im Oberflächenschutz, besonders jedoch zur Herstellung von gehärteten Produkten für elektrische und vor allem elektronische Anwendungen. Sie können in jeweils dem speziellen Anwendungsgebiet angepasster Formulierung, in ungefülltem oder gefülltem Zustand, z.B. als Anstrichmittel, Beschichtungsmassen, Lacke, Pressmassen, Tauchharze, Giessharze, Imprägnierharze, Laminierharze und Klebemittel verwendet werden.

Die Härtung der erfindungsgemässen Gemische kann auf an sich bekannte Weise ein- oder zweistufig vorgenommen werden. Die Härtung der erfindungsgemässen Gemische erfolgt im allgemeinen durch Erhitzen auf Temperaturen zwischen 80 und 200°C, besonders 100 und 180°C.

Die mit den erfindungsgemässen Verbindungen der Formel I hergestellten gehärteten Produkte zeichnen sich durch gute mechanische, thermische und chemische Eigenschaften aus, z.B. hohe Zugscherfestigkeit, gute Wärmeformbeständigkeit und gute chemische Beständigkeit.

Die Erfindung wird durch die folgenden Beispiele näher erläutert.

A. Herstellung des Ausgangsprodukts (4-Allyl-2,6-dimethylphenylglycidylether)

In einem Reaktionsgefäss aus Glas, versehen mit Dosiervorlage, Rührer, Thermometer und einem Azeotropdestillationsaufsatz mit Rückflusskühler und Vakuumanschluss werden 350 g (1 Mol) 4-Allyl-2,6-dimethylphenol, 1796 g (19,42 Mol) Epichlorhydrin und 16,20 g Tetramethylammoniumchlorid zusammengemischt, auf 60°C erwärmt und 2 Stunden bei 60°C gerührt. Durch Anlegen eines Vakuums von 6700 Pa wird das Epichlorhydrin bei der Reaktionstemperatur (ca. 60°C) zum Rückfluss gebracht. Im Verlaufe von 3,5 Std. werden tropfenweise 190 g einer 50%igen wässrigen NaOH-Lösung zugegeben, wobei die Innentemperatur bei 60°C gehalten wird. Während der Reaktion wird das gebildete Wasser mit dem siedenden Epichlorhydrin abdestilliert. Nach der Zugabe der Natronlauge wird das Reaktionsgemisch noch etwa 2 Std. weitergerührt. Nach der Beendigung der Reaktion wird das gebildete Kochsalz abfiltriert, und das Filtrat wird mit 1 Liter 10%iger $Na_2SO_4$-Lösung neutralisiert. Die organische Phase wird abgetrennt und zweimal mit je 600 ml destilliertem Wasser gewaschen, von der wässrigen Phase getrennt und mit Natriumsulfat getrocknet. Anschliessend wird das überschüssige Epichlorhydrin mit Hilfe eines Rotationsverdampfers bei 60°C/2660 Pa abdestilliert. Die Destillation des Rückstands bei 110°C/1,3 Pa ergibt 358 g (75,5 % d. Th.)4-Allyl-2,6-dimethylphenyglycidylether; Epoxidgehalt 4,54 Aequivalente/kg.

| | | |
|---|---|---|
| Elementaranalyse: berechnet | C 76,68% | H 8,73% |
| gefunden | C 76,52% | H 8,43% |

$^1$H-NMR-Spektrum: 2,2 ppm (s) 6H ($CH_3$), ca. 2,75 ppm (m) 2H 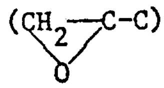,

ca. 3,3 ppm (m) 3H 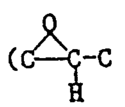 und 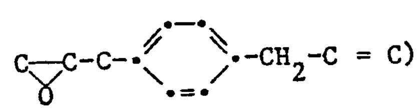,

ca. 3,85 ppm (m) 2H 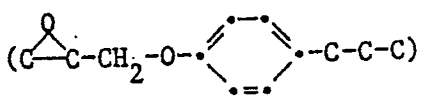, 5,1 ppm (m) 2H

$(CH_2{=}C{-}C)$, 6,0 ppm (m) 1H $(C{=}\underset{\overset{|}{H}}{C}{-}C)$,

6,85 ppm (m) 2H 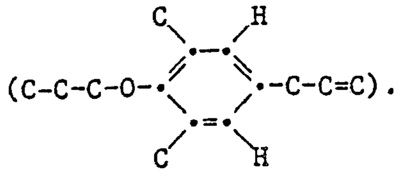

B. Herstellbeispiele

Beispiel 1

In einem 350 ml Sulfierkolben, der mit Thermometer, Rührer, Kühler und Tropftrichter ausgestattet ist, werden 46,30 g (0,21 Mol) 4-Allyl-2,6-dimethylphenylglycidylether, 1,93 g Natriumacetat und 160 ml Chloroform vorgelegt und auf 35—40°C erwärmt. Anschliessend werden bei dieser Temperatur innerhalb von etwa 1 Stunde 52,3 g (0,28 Mol) 40%ige Peressigsäure zugetropft. Das Reaktionsgemisch wird während weiterer 4 Stunden bei dieser Temperatur gehalten, bis im Gaschromatograph kein Edukt mehr zu ermitteln ist. Nach Beendigung der Reaktion wird das Reaktionsgemisch in Chloroform verdünnt, mit 3%iger NaOH neutral gewaschen, mit Natriumsulfit peroxidfrei gestellt, über Natriumsulfat getrocknet, filtriert und am Rotationsverdampfer eingeengt. Man erhält 47,6 g 4-(2,3-Epoxypropyl)-2,6-dimethylphenylglycidylether in Form eines niedrig viskosen Oels; Epoxidgehalt 8,1 Aequivalent/kg (95,13% d. Th.).

$^1$H-NMR-Spektrum: 2,25 ppm (s) 6H $(CH_3)$, 2,5—3,0 ppm (m) 6H

$^1$H-NMR-Spektrum: 2,25 ppm (s) 6H $(CH_3)$, 2,5-3,0 ppm (m) 6H

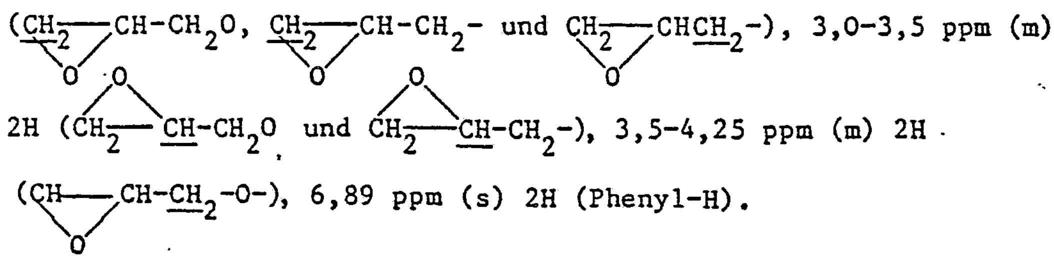
und , 3,0-3,5 ppm (m)
2H und , 3,5-4,25 ppm (m) 2H
, 6,89 ppm (s) 2H (Phenyl-H).

Beispiel 2

In einem 350 ml Sulfierkolben, der mit Thermometer, Rührer, Kühler und Tropftrichter ausgestattet ist, werden 21,8 g (0,10 Mol) 4-Allyl-2,6-dimethylphenylglycidylether, 10,9 g (0,24 Mol) Ameisensäure und 135 ml Chloroform vorgelegt. Anschliessend werden bei Raumtemperatur innerhalb von 4 Std. 23,2 g (0,47 Mol) Wasserstoffperoxid zugetropft. Das Reaktionsgemisch wird Nacht bei dieser Temperatur gehalten. Anschliessend wird es in Chloroform aufgenommen, zweimal mit 3%iger NaOH-Lösung gewaschen, mit Natriumsulfit peroxidfrei gestellt, über Natriumsulfat getrocknet, filtriert und eingeengt. Man erhält 20,03 g (85,50% d.Th) 4-(2,3-Epoxypropyl)-2,6-dimethylphenylglycidylether in Form eines niedrig viskosen Harzes; Epoxidgehalt 7,7 Aequivalente/kg (90,11% d. Th.).

Beispiel 3

4-(2,3-Epoxypropyl)-2,6-Dichlorphenylglycidyläther

In einem 100 ml Sulfierkolben mit Rührer, Kühler, Thermometer und Tropftrichter werden 13,0 g (0,05 Mol) 4-Allyl-2,6-Dichlorophenylglycidyläther in 40 ml Toluol und 0,5 g Natriumacetat vorgelegt. Innerhalb 2 Std. werden bei 30—50°C 14,0 g (0,07 Mol) 10%ige Peressigsäure zugetropft.

Nach Zutropfen wird das Reaktionsgemisch mit 300 ml Toluol verdünnt, die entstandene wässrige Phase wird abgetrennt, die organische Phase wird 2x mit 150 $NaHCO_3$ (5%) gewaschen, mit 150 ml Wasser nachgewaschen, die organische Phase wird über Natriumsulfat und Natriumsulfit getrocknet bzw. Peroxydfrei gestellt, filtriert und eingeengt.

Es resultieren 12,7 g (92,3%) eines hellgelben Harzes vom Epoxydgehalt 5,62 eq/kg (77,75%) und einer Viskosität von 280 mPas/25°C.

Beispiel 4

4-(2,3-Epoxypropyl)-2,6-Diphenylphenylglycidyläther

In einem 100 ml Sulfierkolben mit Rührer, Kühler, Thermometer und Tropftrichter werden 13,7 g (0,04 Mol) 4-Allyl-2,6-Diphenylphenylglycidyläther in 20 ml Toluol und 0,7 g Natriumacetat vorgelegt. Innerhalb von 2 Std. werden zwischen 30—50°C 9,13 g (0,048 Mol) 40%ige Peressigsäure zugetropft. Nach Beendigen des Zutropfens wird das Reaktionsgemisch noch während 7 Std. gerührt.

Nach Beendigen der Reaktion wird das Reaktionsgemisch mit 700 ml Toluol verdünnt, die entstandene wässerige Phase wird abgetrennt, die organische Phase wird 2x mit 150 ml $NaHCO_3$ (5%) und 150 ml

Wasser gewaschen, die organische Phase über $Na_2SO_4$ und $Na_2SO_3$ getrocknet und peroxydfrei gestellt, filtriert une eingeengt. Es resultieren 12,46 g (86,5%) eines gelben Harzes vom Epoxydäquivalent 4,99 eq/kg (89,5%) und einer Viskosität von 6850 mPas/40°C.

C. Verwendungsbeispiele

Beispiele I und II

Die folgenden Bestandteile werden zu härtbaren Gemischen A und B verarbeitet (Gewichtsteile):

| | Gemisch A | Gemisch B |
|---|---|---|
| 4-(2,3-Epoxypropyl)-2,6-dimethyl-phenylglycidylether | 100 | 100 |
| 4,4'-Diaminodiphenylmethan (Härter) | 39,6 | — |
| Hexahydrophthalsäureanhydrid (Härter) | — | 104,5 |
| 3-Methylimidazol (Beschleuniger) | — | 0,5 |

Von diesen Gemischen werden die Reaktivität mittels der Gelierzeiten und die Viskositäten bestimmt. Die Resultate sind in der folgenden Tabelle I zusammengefasst.

Tabelle 1

| Beispiel | | | I (Gemisch A) | II (Gemisch B) |
|---|---|---|---|---|
| Gelierzeiten bei | 100°C | min/sec | n.b. | 100' |
| | 120°C | | 50' | 26' |
| | 140°C | | 25'15" | 8'13" |
| | 160°C | | 12'47" | 3' |
| | 180°C | | 5'43" | 1'21" |
| Viskosität η bei 80°C: | | | | |
| Anfangsviskosität | | mPas | 15 | 10 |
| Viskosität verdoppelt nach | | min | ca. 110 | 80 |

n.b. = nicht bestimmt

Beispiele III—VI

Die in den Beispielen I und II beschriebenen Gemische A und B werden zu Formkörpern bzw. Filmen verarbeitet und
1) während 4 Stunden bei 100°C und 8 Stunden bei 140°C bzw.
2) während 6 Stunden bei 180°C gehärtet.

An den gehärteten Formkörpern bzw. Filmen werden die folgenden Eigenschaften bestimmt:
die Glasumwandlungstemperatur Tg:
bestimmt mittels DSC (Differential Scanning Calorimetry)
bestimmt mittels thermomechanischer Analyse (TMA)
die Zugscherfestigkeit nach DIN 53 283
die chemische Beständigkeit und die Reibfestigkeit nach DIN 53 230 (an Filmen).
Die Resultate sind in der folgenden Tablelle II zusammengefasst.

Tabelle II

| Beispiel | | I,II (Gemisch A) | IV (Gemisch B) | V (Gemisch A) | VI (Gemisch B) |
|---|---|---|---|---|---|
| Härtung bei | | 4 Std./100°C + 8 Std./ 140°C | | 6 Std./180°C | |
| Tg bestimmt mittels DSC | °C | 163 | 120 | 177 | 130 |
| bestimmt mittels TMA | °C | 159 | 118 | 168 | 119 |
| Zugscherfestigkeit nach DIN 53 283 1) | $N/mm^2$ | 9,3 | 13,1 | 10,3 | 15,7 |
| chemische Beständigkeit nach DIN 53 230: | | | | | |
| in destilliertem Wasser | | 0 | n.b. | 0 | n.b. |
| in 5N NaOH | | 0 | n.b. | 0 | n.b. |
| in 5N $H_2SO_4$ | | 0 | n.b. | 0 | n.b. |
| Reibtest nach DIN 53 230 (20 Reibungen) | 2) | | | | |
| in Aceton | | 0 | n.b. | 0 | n.b. |
| in Chlorbenzol | | 0 | n.b. | 0 | n.b. |

1) Durchschnitt von 10 Messungen

2) Der Wert "0" bedeutet, dass nach 20 Hin- und Herreibungen mit einem mit den genannten Lösungsmitteln getränkten Wattebausch keine Veränderung der Filmoberfläche beobachtet wird.

n.b. = nicht bestimmt.

**Patentansprüche**

1. Verbindungen der Formel I

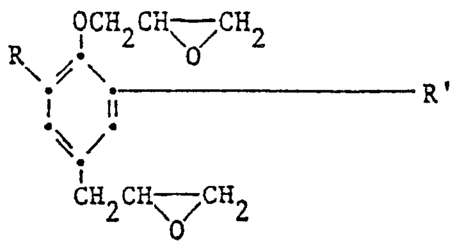

(I),

worin R und R′ unabhängig voneinander $C_{1-4}$-Alkyl, $C_{1-4}$-Alkoxy ein Halogenatom oder $C_{6-10}$-Aryl bedeuten.

2. Verbindungen der Formel I nach Anspruch 1, worin R und R′ unabhängig voneinander $C_{1-4}$-Alkyl, $C_{1-2}$-Alkoxy, ein Halogenatom, besonders ein Chloratom, oder Phenyl bedeuten.

3. Verbindungen der Formel I nach Anspruch 1, worin R und R′ dieselbe Bedeutung haben.

4. Verbindungen der Formel I nach Anspruch 1, worin R und R′ je Methyl, tert-Butyl, Methoxy, Chlor oder Phenyl bedeuten.

5. Verbindungen der Formel I nach Anspruch 4, worin R und R′ je Methyl bedeuten.

6. Verfahren zur Herstellung von Verbindungen der Formel I nach Anspruch 1, dadurch gekennzeichnet, dass man einen Allylphenylglycidylether der Formel II

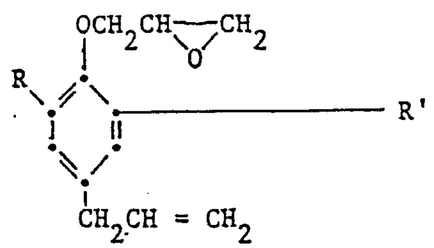

(II),

worin R und R′ unabhängig voneinander $C_{1-4}$-Alkyl, $C_{1-4}$-Alkoxy ein Halogenatom oder $C_{6-10}$-Aryl darstellen, in Gegenwart einer Persäure epoxidiert.

7. Verfahren nach Anspruch 6, dadurch gekennzeichnet, dass man die Epoxidierung in Gegenwart von Perameisensäure oder Peressigsäure vornimmt.

8. Härtbare Gemische enthaltend
(a) eine Verbindung der Formel I nach Anspruch 1,
(b) einen Härter für die Komponente (a).

9. Härtbare Gemische nach Anspruch 8, die zusätzlich (c) einen Härtungsbeschleuniger enthalten.

10. Härtbare Gemische nach Anspruch 8 oder 9, die zusätzlich (d) ein weiteres Epoxidharz enthalten.

11. Verwendung von härtbaren Gemischen nach Anspruch 8 als Klebstoffe oder im Oberflächenschutz und insbesondere zur Herstellung von gehärteten Produkten für elektrische und elektronische Anwendungen.

**Revendications**

1. Composés répondant à la formule I:

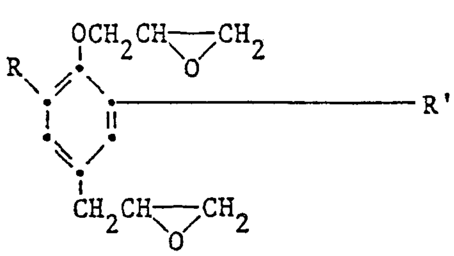

(I),

dans laquelle R et R' représentent chacun, indépendamment l'un de l'autre, un alkyle en $C_1$—$C_4$, un alcoxy en $C_1$—$C_4$, un atome d'halogène ou un aryle en $C_6$—$C_{10}$.

2. Composés de formule I selon la revendication 1 dans lasquels R et R' représentent chacun, indépendamment l'un de l'autre, un alkyle en $C_1$—$C_4$, un alcoxy en $C_1$ ou $C_2$, un atome d'halogène, en particulier de chlore, ou un phényle.

3. Composés de formule I selon la revendication 1 dans lesquels R et R' ont la même signification.

4. Composés de formule I selon la revendication 1 dans lesquels R et R' représentent chacun un méthyl, un tert-butyle, un méthoxy, le chlore ou un phényle.

5. Composé de formule I selon la revendication 4 dans lequel R et R' représentent chacun un méthyle.

6. Procédé pour préparer des composés de formule I selon la revendication 1, procédé caractérisé en ce qu'on époxyde en présence d'un peracide un oxyde de glycidyle et d'allylphényle répondant à la formule II:

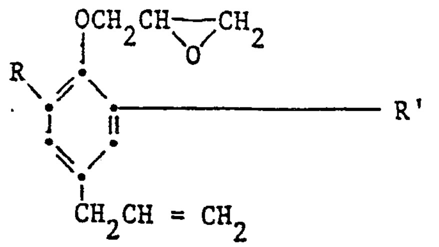

(II),

dans laquelle R et R' représentent chacun, indépendamment l'un de l'autre, un alkyle en $C_1$—$C_4$, un alcoxy en $C_1$—$C_4$, un atome d'halogène ou un aryle en $C_6$—$C_{10}$.

7. Procédé selon la revendication 6 caractérisé en ce qu'on effectue l'époxydation en présence d'acide performique ou d'acide peracétique.

8. Mélanges durcissables qui contiennent:

(a) un composé de formule I selon la revendication 1, et

(b) un durcisseur pour la composante (a).

9. Mélanges durcissables selon la revendication 8 qui contiennent en outre (c) un accélérateur de durcissement.

10. Mélanges durcissables selon l'une des revendications 8 et 9 qui contiennent en outre (d) une résine époxydique supplémentaire.

11. Application de mélanges durcissables selon la revendication 8 comme colles ou dans la protection de surfaces, et plus particulierement pour la fabrication de produits durcis destinés à des applications électriques et électroniques.

## Claims

1. A compound of formula I

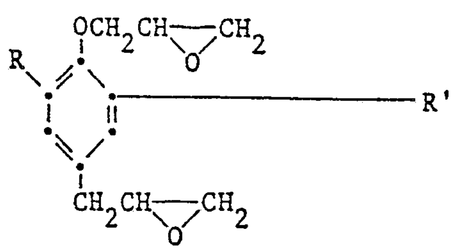

(I),

wherein each of R and R' independently of the other is $C_1$—$C_4$ alkyl, $C_1$—$C_4$ alkoxy, a halogen or $C_6$—$C_{10}$ aryl.

2. A compound of formula I according to claim 1, wherein each of R and R' independently of the other is $C_1$—$C_4$ alkyl, $C_1$—$C_2$alkoxy, a halogen atom, in particular a chlorine atom, or phenyl.

3. A compound of formula I according to claim 1, wherein each of R and R' have the same meaning.

4. A compound of formula I according to claim 1, wherein each of R and R' is methyl, tert-butyl, methoxy, chlorine or phenyl.

5. A compound of formula I according to claim 4, wherein each of R and R' is methyl.

6. A process for the preparation of a compound of formula I according to claim 1, which process comprises epoxidizing an allylphenyl glycidyl ether of formula II

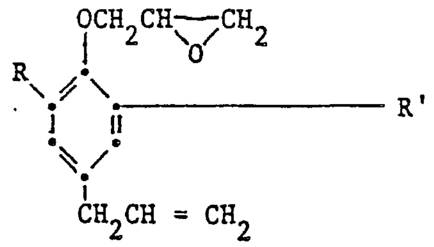

(II),

wherein each of R and R′ independently of the other is $C_1$—$C_4$ alkyl, $C_1$—$C_4$ alkoxy, a halogen atom or $C_6$—$C_{10}$ aryl, in the presence of a peracid.

7. A process according to claim 6, which comprises carrying out the epoxidation in the presence of performic acid or peracetic acid.

8. A curable mixture containing (a) a compound of formula I according to claim 1 and (b) a hardener for the component (a).

9. A curable mixture according to claim 8, which additionally contains (c) a curing accelerator.

10. A curable mixture according to either of claims 8 or 9, which additionally contains (d) a further epoxy resin.

11. Use of a curable mixture according to claim 8 as adhesive or in surface protection and, in particular, for the preparation of cured products for electrical and electronic applications.